# EUROPEAN PATENT SPECIFICATION

(11) **EP 4 732 893 B1**
(45) Date of publication and mention of the grant of the patent: **02.12.2026**
(21) Application number: 25210468.2
(22) Date of filing: 22.10.2025
(51) Int. Cl.: A61N 1/37, A61B 5/00

(54) **BIOSTIMULATOR HAVING A STIFFENED PACING ELEMENT**
BIOSTIMULATOR MIT VERSTEIFTEM SCHRITTMACHERELEMENT
BIOSTIMULATEUR AYANT UN ÉLÉMENT DE STIMULATION RIGIDIFIÉ

(30) Priority: 23.10.2024 US 202463711107 P; 20.10.2025 US 202519363517
(43) Date of publication of application: 29.04.2026
(73) Proprietor: Pacesetter, Inc., Sylmar, CA 91342 (US)
(72) Inventor: Teague, Bryan, Sylmar, CA 91342 (US); Chantasirivisal, Steve, Sylmar, CA 91342 (US); Victorine, Keith, Sylmar, CA 91342 (US); Kwon, Daniel, Sylmar, CA 91342 (US); Manalo, Megan, Sylmar, CA 91342 (US); Welch, Mark, Sylmar, CA 91342 (US)
(74) Representative: Barker Brettell Sweden AB

(56) References cited:
- EP-A2- 4 197 589
- WO-A1-2016/172106
- US-A1- 20240 009 468
- US-B2- 10 674 928

## Description

### TECHNICAL FIELD

The present disclosure relates to biostimulators. More specifically, the present disclosure relates to leadless biostimulators useful for deep septal pacing.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often lengthy and requires significant fluoroscopic exposure. Furthermore, successful His-bundle pacing cannot always be achieved. Pacing thresholds are often high, sensing is challenging, and success rates can be low.

Deep septal pacing is an alternative to His-bundle pacing. Deep septal pacing involves pacing past the His-bundle toward the right ventricle apex. More particularly, deep septal pacing targets the left bundle branch below the His site. Deep septal pacing has been achieved using a lead in which the electrode penetrates several millimeters into the septum. Pacing thresholds associated with deep septal pacing are potentially lower than with His-bundle pacing, and clinical efficacy of the approach has been demonstrated.

US 10,674,928 B2 discloses a leadless pacing system including a leadless pacing device (LPD) and a sensing extension extending form a housing of the LPD. The sensing extension includes at least one electrode with which the LPD may sense electrical cardiac activity. The at least one electrode of the sensing extension is carried by a self-supporting body that passively positions the at least one electrode proximate or within a chamber of the heart other than the chamber in which the LPD is implanted.

### SUMMARY

Existing pacing leads are not well suited to pacing the deep septal area. Deep septal pacing, e.g., left bundle branch area pacing (LBBAP), can require a pacing electrode to penetrate through a majority of a ventricular septal wall to extend into the left bundle branch or into a left bundle fascicular that resides on a left side of a septum. More particularly, the pacing electrode may be required to penetrate 10-12 mm into the ventricular septal wall. Existing pacing leads lack the ability to control penetration into the target tissue and are susceptible to buckling. Thus, the leads may not penetrate beyond the fibrous endocardial tissue. In addition to the stiffness required to achieve deep penetration, an effective LBBAP lead may require resistance against fatigue failure caused by many bending cycles. Accordingly, there is a need for a leadless biostimulator having a pacing element configured to reach the deep septal area without buckling for deep septal pacing of a heart, and which can withstand fatigue throughout an operational life.

A biostimulator is described. The biostimulator includes a housing having a longitudinal axis and containing an electronics compartment. The biostimulator includes a fixation element coupled to a distal housing end of the housing. The fixation element extends about the longitudinal axis and includes a helical fixation element. The biostimulator includes a pacing element coupled to the distal housing end of the housing. The pacing element includes a flexible conductor including a conductive cable extending beside the longitudinal axis, wherein the conductive cable is a conductive coil. The pacing element also includes a helical electrode coupled to the flexible conductor, an insulative sleeve surrounding the pacing element, and a plurality of stiffening wires disposed radially about the flexible conductor. The plurality of stiffening wires forms a stiffening braid extending through a wall of the insulative sleeve. A bending stiffness of the stiffening braid is greater than a bending stiffness of the flexible conductor.

A biostimulator system is described. The biostimulator system includes a biostimulator transport system, and the biostimulator referred to above mounted on the biostimulator transport system.

The above summary does not include an exhaustive list of all aspects of the present description. It is contemplated that the present teaching includes all systems and methods that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments of the present teaching in which the principles of the invention are utilized, and the accompanying drawings.
FIG. 1 is a diagrammatic cross section of a patient heart illustrating an example implantation of a biostimulator in a target anatomy, in accordance with an embodiment.
FIG. 2 is a perspective view of a biostimulator system, in accordance with an embodiment.
FIG. 3 is a side view of a biostimulator having a pacing element, in accordance with an embodiment.
FIG. 4 is an end view of a biostimulator having a pacing element, in accordance with an embodiment.
FIG. 5 is a side view of a distal portion of a biostimulator having a stiffened pacing element, in accordance with an embodiment.
FIG. 6 is a side view of a distal portion of a biostimulator having a stiffened pacing element, in accordance with an embodiment.
FIG. 7 is a side view of a stiffening element, in accordance with an embodiment.
FIG. 8 is a side view of a distal portion of a biostimulator having a stiffened pacing element, in accordance with an embodiment.
FIG. 9 is a rear perspective view of a distal portion of a biostimulator having a stiffened pacing element, in accordance with an embodiment.
FIG. 10 is a side view of a distal portion of a biostimulator having a stiffened pacing element, in accordance with an embodiment.
FIG. 11 is a perspective view of a header assembly of a biostimulator system, in accordance with an embodiment.
FIG. 12 is a cross-sectional view of a header assembly of a biostimulator system, in accordance with an embodiment.
FIG. 13 is a cross-sectional view of a pacing element of a biostimulator system, in accordance with an embodiment.
FIG. 14 is a cross-sectional view of a pacing element of a biostimulator system, in accordance with an embodiment.

### DETAILED DESCRIPTION

Embodiments describe a biostimulator, such as a leadless pacemaker, having a stiffened pacing element. As described below, the biostimulator can be used to perform deep septal pacing of a heart. The biostimulator may, however, be used in other applications, such as deep brain stimulation. Thus, reference to the biostimulator as being a cardiac pacemaker for deep septal pacing is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a biostimulator. Similarly, "proximal" may indicate a second direction opposite to the first direction. Such terms are provided to establish relative frames of reference, however, and are not intended to limit the use or orientation of a biostimulator to a specific configuration described in the various embodiments below.

In an aspect, a biostimulator for deep septal pacing includes a stiffened pacing element. As described above, the optimal pacing element may both resist buckling during tissue penetration and flex during operation to resist fatigue failure. Such mechanical characteristics typically counteract each other (stiffer members typically have a shorter fatigue life, while less stiff members typically have a longer fatigue life). The described biostimulator include a pacing element that includes a stiffening member, e.g., a stiffening strand or a stiffening wire, to augment a flexible conductor. The flexible conductor can carry pacing impulses to an electrode, and may be highly flexible. The stiffening member may have a higher bending stiffness than the flexible conductor. The higher bending stiffness can resist buckling and support penetration of a target tissue by the pacing element. A resilience of the stiffening member may provide a strain relief to support cyclic loading of the pacing element and resist fatigue. Accordingly, the stiffened pacing element both resists buckling and fatigue.

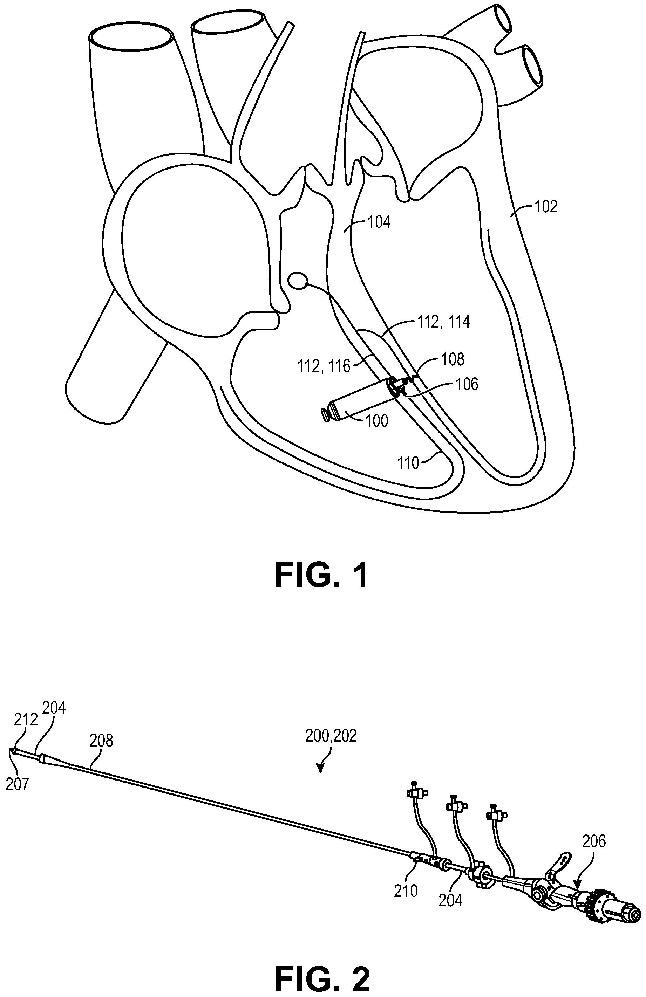

Referring to FIG. 1, a diagrammatic cross section of a patient heart illustrating an example implantation of a biostimulator in a target anatomy is shown in accordance with an embodiment. A leadless biostimulator system, e.g., a cardiac pacing system, includes one or more biostimulators 100. The biostimulators 100 can be implanted in a patient heart 102, and can be leadless (and thus, may be leadless cardiac pacemakers). Each biostimulator 100 can be placed in a cardiac chamber, such as a right atrium and/or right ventricle of the heart 102, or attached to an inside or outside of the cardiac chamber. For example, the biostimulator 100 can be attached to a septum 104 of the heart 102. More particularly, the biostimulator 100 can be delivered to the septum 104, and one or more elements, such as a fixation element 106 and/or a pacing element 108 can pierce a septal wall 110 of the septum 104 to engage and anchor the biostimulator 100 to the tissue. In a particular embodiment, the biostimulator 100 can use two or more electrodes located on or within a housing of the biostimulator 100 for pacing the cardiac chamber upon receiving a triggering signal from at least one other device within the body. In an embodiment, one or more of the fixation element 106 or the pacing element 108 is an active electrode.

Leadless pacemakers or other leadless biostimulators 100 can be delivered to or retrieved from a patient using delivery or retrieval systems. The leadless biostimulator system can include delivery or retrieval systems, which may be catheter-based systems used to carry a leadless biostimulator 100 intravenously to or from a patient anatomy. The delivery or retrieval systems may be referred to collectively as transport systems. In some implementations of transport systems, a leadless pacemaker is attached or connected to a distal end of a catheter and advanced intravenously into or out of the heart 102. The transport system can include features to engage the leadless pacemaker to allow fixation of the leadless pacemaker to tissue. For example, in implementations where the leadless pacemaker includes an active engaging mechanism, such as a fixation element 106, the transport system can include a docking cap or key at a distal end of the catheter, and the docking cap or key may be configured to engage the leadless pacemaker and apply torque to screw the active engaging mechanism into or out of the tissue. In other implementations, the transport system includes clips designed to match the shape of a feature on the leadless pacemaker and apply torque to screw the active engaging mechanism into or out of the tissue.

When the biostimulator 100 is delivered to and screwed into the septum 104 of the heart 102, the pacing element 108 and/or the fixation element 106 may be positioned for deep septal pacing at respective bundle branches 112 in the septum 104. For example, the biostimulator 100 can be deployed to a mid-septum with sufficient depth such that the pacing element 108 can be positioned at the left bundle branch 114 in the septum 104. The pacing element 108 can be an active electrode to stimulator the left ventricle by pacing the left ventricular conduction system, e.g., the left bundle branch 114, fascicles, or Purkinje fibers. Similarly, the fixation element 106 can be positioned to stimulate the right ventricle. For example, the fixation element 106 can be positioned at the right bundle branch 116 in the septum 104. The fixation element 106 may be an active electrode to stimulate the right ventricle by pacing the right ventricular conduction system, e.g., the right bundle branch 116, fascicles, or Purkinje fibers. Accordingly, the pacing element 108 may provide left ventricle pacing and the fixation element 106 may provide right ventricle pacing to achieve the benefit of dual ventricular pacing with a single leadless biostimulator 100. Optionally, one of the elements may be at a bundle branch and the other element may not be at a bundle branch.

Referring to FIG. 2, a perspective view of a biostimulator system is shown in accordance with an embodiment. A biostimulator system 200 includes the biostimulator 100 (not shown) mounted on a biostimulator transport system 202. As described above, the biostimulator 100 can be delivered to and retrieved from a patient anatomy using the biostimulator transport system 202. In some implementations, the biostimulator transport system 202 is a delivery system for delivering the leadless pacemaker to the target tissue. In some implementations, the biostimulator transport system 202 is a retrieval system for retrieving the leadless pacemaker from the target tissue. The biostimulator transport system 202 can include a release mechanism to retain any of the biostimulators described below in an unreleased state and to transition into a released state to release the biostimulators into the target anatomy.

The biostimulator transport system 202 can include an elongated catheter 204 extending distally from a handle 206 to a distal catheter end 207. The elongated catheter 204 can be a deflectable catheter, and an operator can use the handle 206 to steer the distal catheter end 207 in the patient. In an embodiment, the biostimulator transport system 202 includes a guide catheter 208 mounted on the elongated catheter 204. The guide catheter 208 can be slidably disposed on the elongated catheter 204 such that a distal portion of the guide catheter 208 can slide distally over the distal catheter end 207 of the elongated catheter 202 and/or the biostimulator 100, which may be mounted on the distal catheter end (not shown). Similarly, the biostimulator transport system 200 can include an introducer hub assembly 210 mounted on the guide catheter 208. The introducer hub assembly 210 can be slidably disposed on the guide catheter 208 such that a distal portion of the introducer hub assembly 210 can slide distally over the distal catheter end 207 of the elongated catheter 204 and/or the distal portion of the guide catheter 208. More particularly, the introducer hub assembly 210 can be inserted into an access sheath to gain access to the patient vasculature, and after access is established, the distal portion of the guide catheter 208 and/or the distal catheter end 207 of the elongated catheter 204 can be advanced through the access sheath into the patient.

The distal catheter end 207 of the elongated catheter 204 may be selectively connectable to the biostimulator 100. More particularly, the biostimulator 100 can be mounted on the distal catheter end 207 of the elongated catheter 204. The biostimulator 100 can be protected by a protective sheath of the distal portion of the guide catheter 208 during delivery and/or retrieval of the biostimulator 100 from the patient. Accordingly, the biostimulator 100 can be advanced into the patient along with the distal catheter end 207.

The leadless pacemaker system can be used to implant one or more biostimulators 100 within an atrium and/or a ventricle of a heart 102 of the patient. Implantation of each biostimulator 100 may be achieved, in part, by endocardial insertion of the biostimulators 100. For example, the elongated catheter 204 of the leadless pacemaker system can include a torque shaft coupled to a docking cap 212. The docking cap 212 can have a docking cavity to receive an attachment feature of the biostimulator 100. The torque shaft can be torqueable and rotation of the torque shaft can rotate the docking cap 212, which can impart rotation to the attachment feature. Accordingly, torque can be transmitted through the torque shaft to rotate the biostimulator 100 in a first direction, e.g., clockwise. Rotating the biostimulator 100 when a fixation element 106 is in contact with the heart tissue can cause the fixation element to screw into the heart tissue and affix the biostimulator 100 to the heart tissue. Similarly, removal and retrieval of the biostimulators 100 may be accomplished endocardially. For example, the torque shaft of the elongated catheter 204 can be rotated in a second direction, e.g., counterclockwise, to transmit torque through the docking cap 212 to the attachment feature to disengage the biostimulator 100 from the heart tissue.

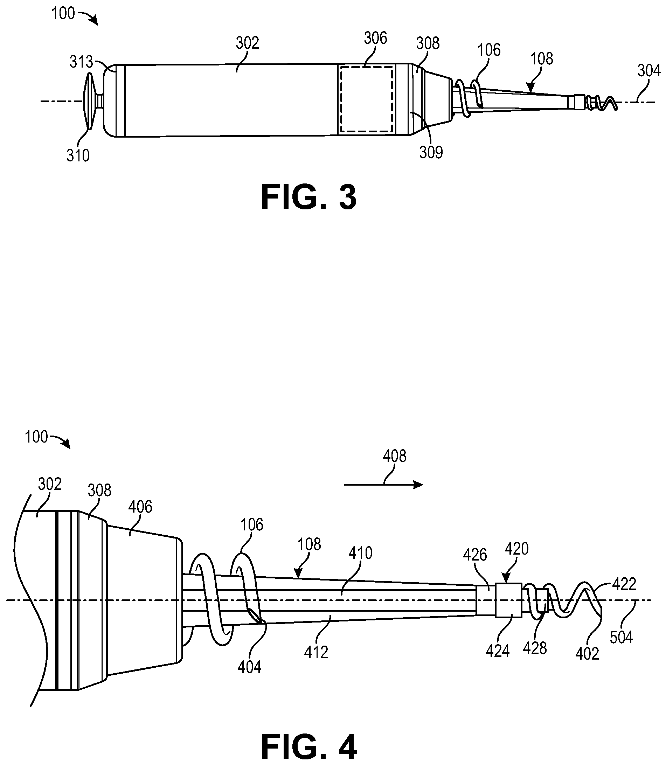

Referring to FIG. 3, a side view of a biostimulator having a pacing element is shown in accordance with an embodiment. The biostimulator 100 can be a leadless cardiac pacemaker that can perform cardiac pacing and that has many of the advantages of conventional cardiac pacemakers while extending performance, functionality, and operating characteristics. The biostimulator 100 can have two or more electrodes, e.g., a portion of a pacing element 108 that acts as an active electrode and/or a portion of the fixation element 106 or a housing 302 that acts as an active electrode. The electrodes can deliver pacing pulses to bundle branches 112 within the septum 104 of the heart 102 to perform deep septal pacing, and optionally, can sense electrical activity from the muscle. The electrodes may also communicate bidirectionally with at least one other device within or outside the body.

In an embodiment, the biostimulator 100 includes the housing 302 having a longitudinal axis 304. The housing 302 can contain a primary battery to provide power for pacing, sensing, and communication, which may include, for example, bidirectional communication. The housing 302 can optionally contain an electronics compartment 306 (shown by hidden lines) to hold circuitry adapted for different functionality. For example, the electronics compartment 306 can contain circuits for sensing cardiac activity from the electrodes, circuits for receiving information from at least one other device via the electrodes, circuits for generating pacing pulses for delivery to tissue via the electrodes, or other circuitry. The electronics compartment 306 may contain circuits for transmitting information to at least one other device via the electrodes and can optionally contain circuits for monitoring device health. The circuit of the biostimulator 100 can control these operations in a predetermined manner. The biostimulator 100 can perform leadless pacing without a pulse generator located in the pectoral region or abdomen and/or without an electrode-lead separate from the pulse generator. The biostimulator 100 may also lack a communication coil or antenna, and may not have the substantial battery power required for transmitted communication through a communication coil or antenna.

Leadless pacemakers or other leadless biostimulators 100 can be fixed to an intracardial implant site, e.g., at the septal wall 110, by one or more actively engaging mechanisms or fixation mechanisms, such as a screw or helical member that screws into the myocardium. In an embodiment, the biostimulator 100 includes the fixation element 106 coupled to the housing 302. The fixation element 106 can include a helical fixation element and/or several tines. More particularly, the biostimulator 100 can include a header assembly having a flange 308 coupled to a distal housing end 309 of the housing 302, and the fixation element 106 can be coupled to and extend distal to the flange 308. The fixation element 106 can extend about the longitudinal axis 304. For example, in the case of a helical fixation element, the element can spiral about the longitudinal axis to a distal fixation tip. In the case of a fixation element 106 having several tines, the tines can be arranged about the longitudinal axis and extend to respective distal tips. Accordingly, when the biostimulator 100 is delivered to the target tissue, the distal tip(s) of the fixation element 106 can pierce the tissue and the housing 302 can be rotated or pushed to affix the fixation element 106 to the target tissue.

In an embodiment, the biostimulator 100 includes an attachment feature 310. The attachment feature 310 can be mounted on a proximal housing end 313 of the housing 302. More particularly, the attachment feature 310 can be mounted on an opposite end of the housing 302 from the fixation element 106 and the pacing element 108, which as described above, can be coupled to the distal housing end 309 of the housing 302. The attachment feature 310 can facilitate precise delivery or retrieval of the biostimulator 100. For example, the attachment feature 310 can be formed from a rigid material to allow a transport system to engage the attachment feature 310 and transmit torque and/or axial loads to the attachment feature to plunge the fixation element 106 or the pacing element 108 into the target tissue.

Referring to FIG. 4, an end view of a biostimulator having a pacing element is shown in accordance with an embodiment. The biostimulator 100 can include the pacing element 108 coupled to the housing 302. The pacing element 108 can be coaxially arranged with the fixation element 106 about the longitudinal axis 304. More particularly, the pacing element 108 can extend along, e.g., axially along or helically about, the longitudinal axis 304 at a location that is radially inward from the fixation element 106. The pacing element 108 can extend distally to a distal tip having a distal pacing point 402. The distal pacing point 402 may be distal to the distal fixation tip 404. The pacing element 108 can include a helical element to screw into a target tissue or a conical element to pierce into the target tissue, as described below. Accordingly, when the fixation element 106 screws into or otherwise engages the target tissue, the pacing element 108 can also engage the tissue, and the housing 302 can be advanced and/or rotated to cause the distal pacing point 402 of the pacing element 108 to pierce the tissue and anchor the biostimulator 100.

One or more of the fixation element 106 or the pacing element 108 can be an active electrode, and can electrically communicate with the circuitry contained in the electronics compartment 306. Accordingly, the anchored element(s) can electrically communicate with the tissue and can transmit electrical pulses between the tissue and the circuitry of the biostimulator 100. To facilitate electrical function of the fixation element 106 and/or the pacing element 108, the element(s) may be coated in titanium nitride to reduce a polarization value of the electrode(s). By way of example, the titanium nitride coating may be in a range of 5 to 50 microns, e.g., 13 microns.

The biostimulator 100 can include a fixation element mount assembly that includes the fixation element 106 mounted on a fixation element mount 406. The fixation element mount 406 can be non-conductive. For example, the fixation element mount 406 can be formed from polyether ether ketone (PEEK). The fixation element 106 can include MP35N wire having a wire diameter of 0.4-0.6 mm, e.g., 0.5 mm. The wire may be formed from a metal, such as stainless steel or a nickel cobalt alloy. The wire can be wrapped into several turns to form a helical fixation element. The turns can be screwed into the septum 104 to anchor the biostimulator 100 within the heart 102. The fixation element mount assembly is described in further detail below.

In an embodiment, the pacing element 108, which is coupled to the housing 302, extends in a distal direction 408 to the distal pacing point 402. More particularly, as described below, the pacing element 108 can include a core assembly 410 that extends between the electronics compartment 306 and the distal pacing point 402. The core assembly 410 can include one or more structural members to carry electrical pulses, such as a flexible conductor as described below, and one or more structural members to provide column strength and/or bending stiffness, such as a stiffening member as described below. The core assembly 410 can extend in the distal direction 408 through the fixation element mount 406 and the fixation element 106 to the distal pacing point 402 distal to the distal fixation tip 404.

In an embodiment, the pacing element 108 of the biostimulator 100 includes an insulation sleeve 412 surrounding, e.g., encapsulating, the core assembly 410. More particularly, the insulation sleeve 412 can cover and/or surround the core assembly 410. The insulation sleeve 412 can insulate a length of the pacing element 108. For example, the electrically conductive component(s) of the core assembly 410 can be encapsulated by the insulation sleeve 412 over its length, including over a portion of the core assembly 410 distal to the fixation element 106.

In an embodiment, the pacing element 108 includes a distal tip 420. The distal tip 420 can include a helical electrode 422. The helical electrode 422 can include an elongated screw. More particularly, the pacing element 108 can include the helical electrode 422 extending about the longitudinal axis 304 to the distal pacing point 402.

The pacing element 108 can extend distal to the fixation element mount 406 by a length sufficient for the distal tip 420 of the pacing element 108 to access the target region, e.g., the left bundle branch 114. The left bundle branch 114 may be at a depth of 10-25 mm from the septal wall 110, typically. Accordingly, the pacing element 108 can have a length of 7-20 mm, e.g., 12 mm or 16 mm, from a distal end of the fixation element mount 406 to the distal pacing point 402. Accordingly, the pacing element 108 can provide a means of pacing a target tissue that is distal to tissue being engaged by the fixation element 106. For example, the pacing element 108 can pace the left bundle branch 114 when the fixation element 106 is engaged near a surface of the septal wall 110 and/or near the right bundle branch 116.

The helical electrode 422 may be mounted on a tip support 424. The tip support 424 can include a base 426, which may be bonded to or crimped on to the core assembly 410, as described below. A boss 428 can extend distal to the base 426. The boss 428 may have an outer diameter that is less than an outer diameter of base 426. The boss 428 may be inserted into an inner channel of the distal pacing element, e.g., the helical electrode 422. The helical electrode 422 may be secured to the tip support 424, e.g., by welding the helical electrode to the base 426 or the boss 428. Accordingly, the distal tip 420 can be secured to the core assembly 410 to receive and relay pacing impulses to the target tissue at the distal pacing point 402.

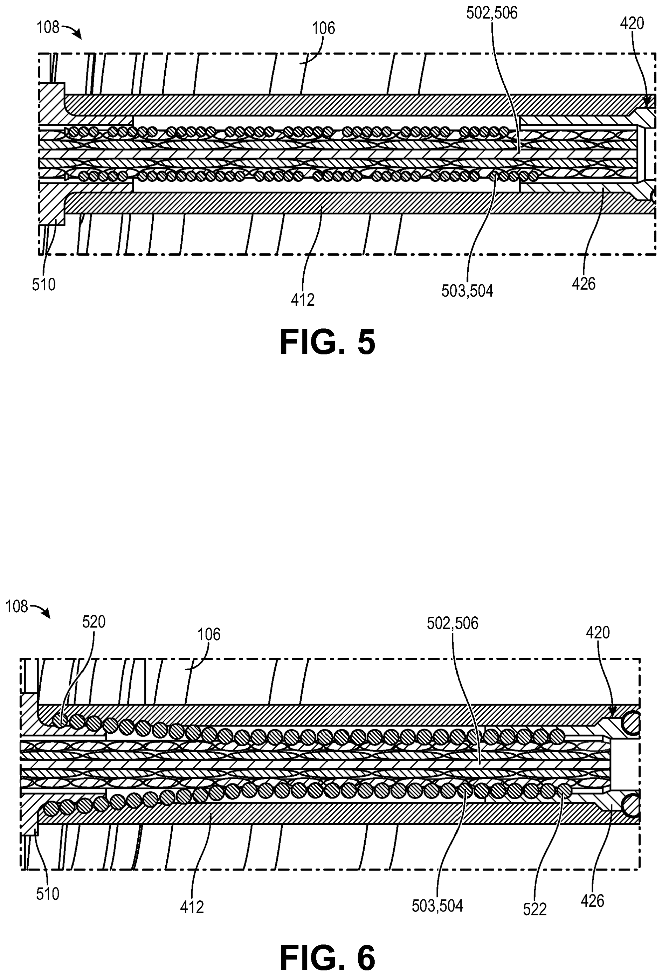

Referring to FIG. 5, a side view of a distal portion of a biostimulator having a stiffened pacing element is shown in accordance with an embodiment. As described above, the core assembly 410 of the pacing element 108 can include one or more electrically active components, such as a flexible conductor 502, and one or more stiffening elements, such as a stiffening strand 503. The stiffening strand 503 may be a component of a stiffening coil 504. More particularly, the stiffening strand 503 may be wound or curved about a central axis, e.g., the longitudinal axis 304, to form the stiffening coil 504. The components can cooperate to provide a pacing structure that can penetrate deeply into the target tissue and resist fatigue stresses, e.g., cyclic bending stresses, which could negatively affect long-term operation of the pacing electrode.

In an embodiment, the flexible conductor 502 can extend along the longitudinal axis 304 between the pacing circuitry and the distal tip 420. More particularly, the flexible conductor 502 may be electrically connected to the distal tip 420 to convey pacing impulses from circuitry within the electronics compartment 306 to the distal pacing point 402. To that end, the flexible conductor 502 can include a conductive cable 506. The conductive cable 506 may include one or more straight or coiled wire strands. For example, the flexible conductor 502 can include a helical conductor wire running along the central axis 304 of the pacing element 108. In an embodiment, the conductive cable 506 includes a stranded cable centrally located and extending along the longitudinal axis 304. The conductive cable 506 may be formed from several wire strands that are coiled or woven to form a flexible cable structure. The flexible conductor 502 may, for example, have a diameter of 0.007-0.040 inch, e.g., 0.010 inch. The cable diameter, angles of the wire strands, and other cable structural characteristics may be adjusted to tune the fatigue resistance of the pacing element 108. High flexibility can allow the conductive cable 506 to experience cyclic bending cycles with a lower likelihood of failure. Accordingly, a bending stiffness may be low, as compared to the stiffening member of the pacing element 108, as described below. The flexible conductor 502 may therefore be optimized for fatigue resistance, signal transmission, and impedance control.

In an embodiment, the flexible conductor 502 is a conductor coil engineered for enhanced fatigue resistance. For example, the flexible conductor 502 can be formed from a fatigueresistant alloy, such as MP35N or platinum-iridium. Furthermore, a coil pitch and diameter may be optimized to balance flexibility and mechanical endurance. Processing operations, such as stressrelief treatments, may also be employed during manufacture of the flexible conductor 502 to improve fatigue life and electrical stability of the conductor.

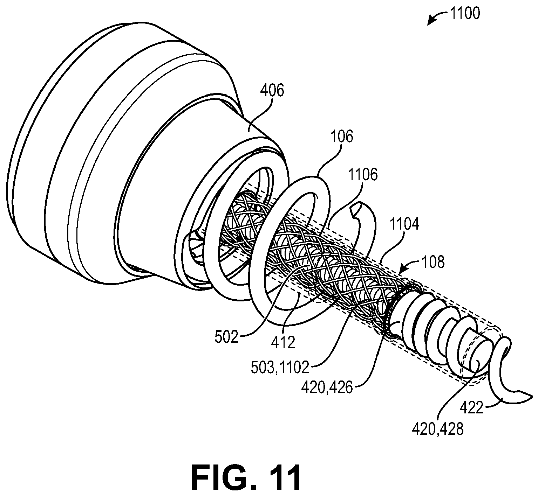

The stiffening member, e.g., the stiffening strand 503, may be electrically inactive. For example, the stiffening coil 504 (or a stiffening braid as described below with respect to FIGS. 11-15) may surround the flexible conductor 502 without actively carrying pacing impulses between the pacing circuitry and the distal tip 420. The stiffening strand 503 (coiled or braided) may, however, have a bending stiffness that is greater than the bending stiffness of the flexible conductor 502. For example, the stiffening strand 503 can be formed from a metal, e.g., MP35N, nickel titanium alloy, platinum-iridium, stainless steel, etc., or a polymer with a higher modulus than a material used to form the flexible conductor 502. Additionally or alternatively, a size and shape of the coiled or braided stiffening strand 503 can be such that the stiffness of the coil or braid under side loads is greater than the flexible conductor 502. Accordingly, the stiffening coil 504 (or braid), which may serve no electrical function, can act as a strain relief to the flexible conductor 502.

In an embodiment, the coiled or braided stiffening strand 503, which has the higher bending stiffness than the flexible conductor 502, may have a lower column strength. More particularly, the flexible conductor 502, which extends along the neutral axis of the pacing element 108, can be axially stiffer than the stiffening coil 504 (or braid). The axial stiffness provided by the flexible conductor 502 can support tissue penetration, and the bending stiffness provided by the stiffening coil 504 (or braid) can support fatigue resistance. Accordingly, the structural component of the pacing element 108 that relieves stress can be physically separated from the structure component that supports tissue penetration.

The braid geometry, e.g., pitch, wire diameter, and carrier count, can be optimized to balance flexibility and mechanical robustness. In an embodiment, the bending stiffness of the stiffening coil 504 (or braid) may vary along a length of the pacing element 108. For example, the bending stiffness can decrease in the distal direction 408. The variation in bending stiffness can result from material or structural variations of the stiffening coil 504 (or braid). For example, a pitch of the coil may decrease in the distal direction 408, resulting in the bending stiffness decreasing in the distal direction 408. More particularly, a decrease in spacing between adjacent turns of the coil can reduce the bending stiffness.

The stiffening strand 503 and/or the flexible conductor 502 may be joined to the distal tip 420. For example, the base 426 of the distal tip 420 can have an inner cavity to receive a distal end of the stiffening coil 504 (or braid) and/or the flexible conductor 502. The base 426 may be crimped, swaged, or otherwise attached to the distal end(s). The helical electrode 422 may therefore be coupled to the flexible conductor 502 via the distal tip 420. Similarly, the stiffening coil 504 (or braid) and/or the flexible conductor 502 may be joined to the fixation element mount 406. For example, a ferrule 510 can be disposed within the fixation element mount 406, and the ferrule 510 may be crimped, swaged, or otherwise attached to the stiffening coil 504 (or braid) and/or the flexible conductor 502. The core assembly 410 can therefore support and connect the distal tip 420 relative to the ferrule 510 and fixation element mount 406.

In an embodiment, the insulation sleeve 412 can include a tubular structure extending distally between the ferrule 510 and the distal tip 420. For example, the insulation sleeve 412 can have a lumen receiving the core assembly 410, and distal and proximal ends of the tubular structure can be mounted on the ferrule 510 and distal tip 420, respectively. Accordingly, an annular wall of the insulation sleeve 412 can surround and contain the core assembly 410. The insulation sleeve 412 can be formed from an electrically insulative material, such as a maleic anhydride modified linear low density polyethylene, a polyurethane, etc. Therefore, the insulation sleeve 412 can, in addition to imparting stiffness, electrically protect a surrounding tissue from electrical pulses carried by the flexible conductor 502 contained within the a lumen of the insulation sleeve 412.

The insulation sleeve 412 may be sized such that a gap is positioned between an inner surface of the annular wall and the stiffening strand 503. The cap can allow the core assembly 410, e.g., the stiffening coil 504 and/or flexible conductor 502, to bend freely within the lumen of the insulation sleeve 412. In an embodiment, however, the gap may be partially or fully filled. For example, the insulation sleeve 412 may be thermally reflowed to collapse around an outer surface of the core assembly 410.

In an embodiment, the core assembly 410 components may be fixed relative to each other. For example, an adhesive may be applied between the flexible conductor 502 and the stiffening coil 504. As a result, the core assembly components can flex as a composite structure. In any case, mechanical stresses can be localized within the stiffening coil 504 to relieve strain of the overall structure. The stiffening coil 504 can flex under such stress, and can release energy after flexure to spring back to a straight configuration. Such loading and unloading can be cyclically applied to the pacing element 108 without resulting in fatigue failure of the core assembly components.

Referring to FIG. 6, a side view of a distal portion of a biostimulator having a stiffened pacing element is shown in accordance with an embodiment. The stiffness transition over a length of the core assembly 410 may result from a taper of the stiffening coil 504. For example, the stiffening coil 504 may taper in a distal direction 408, e.g., from a proximal turn 520 at the ferrule 510 to a distal turn 522 at the distal tip 420. More particularly, a diameter of the stiffening coil 504 can decrease in the distal direction 408. The tapered shape can cause the stiffening coil 504 to have a bending stiffness profile that is more flexible at the distal turn 522 than at the proximal turn 520.

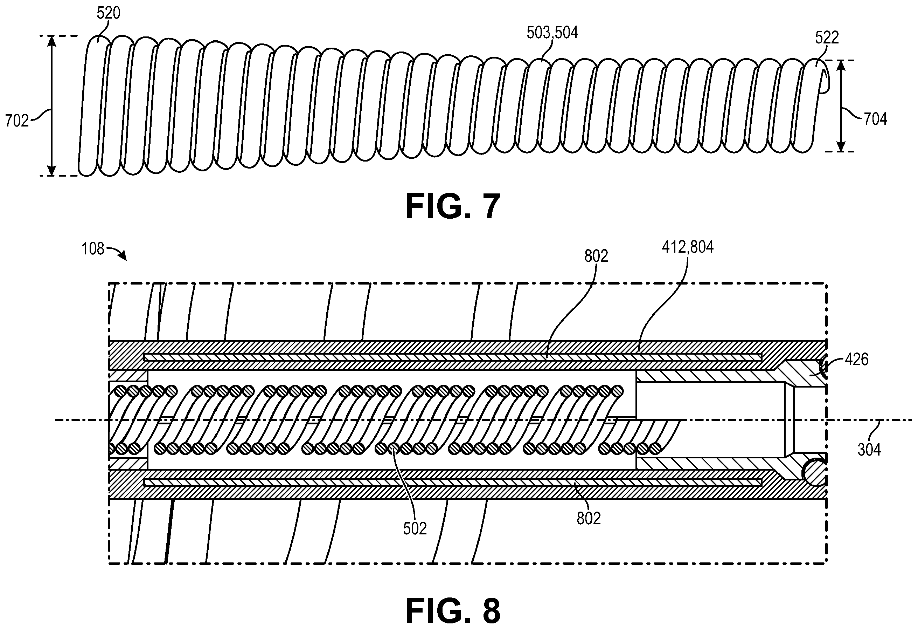

Referring to FIG. 7, a side view of a stiffening element is shown in accordance with an embodiment. The stiffening coil component can have the proximal turn 520 including a first diameter 702. Similarly, the distal turn 522 can have a second diameter 704. The first diameter 702 may be greater than the second diameter 704. Accordingly, the tapered strain relief coil can have a bending stiffness that decreases distally, and the electrically inactive coil can withstand substantial fatigue loading.

Referring to FIG. 8, a side view of a distal portion of a biostimulator having a stiffened pacing element is shown in accordance with an embodiment. The stiffening element of the pacing element 108 may, rather than being coiled, have an elongated structure. For example, the pacing element 108 may be coupled to the housing 302 and include one or more stiffening wires 802 extending longitudinally.

In an embodiment, the pacing element 108 includes the flexible conductor 502 extending along the longitudinal axis 304. For example, the flexible conductor 502 can include a metallic coil extending about, e.g., spiraling around, the longitudinal axis 304. The elongated stiffening wires 802 can extend longitudinally beside the flexible conductor 502. The stiffening wires 802 can extend parallel to the longitudinal axis 304 about which the flexible conductor 502 is coiled. The stiffening wires 802 may be laterally offset from the longitudinal axis 304. For example, the stiffening wires 802 may be radially farther from the longitudinal axis 304 than the flexible conductor 502.

The stiffening wires 802 may have a bending stiffness that is greater than a bending stiffness of the flexible conductor 502. The flexible conductor 502 can be formed from a thin, metallic wire coiled with a tight pitch. Accordingly, the flexible conductor 502 can be highly flexible and have low bending stiffness. By contrast, the stiffening wires 802 may be formed from relatively thick cables, rods, or fibers that resist bending. The stiffening wires 802 may also have high column strength. Accordingly, the stiffening wires 802 can resist fatigue in bending, and can also facilitate tissue penetration by contributing to the column stiffness of the pacing element 108.

The one or more stiffening wires 802 can include several stiffening wires 802 disposed radially about the flexible conductor 502. For example, the pacing element 108 can include two or more stiffening wires 802, e.g., four stiffening wires 802, uniformly distributed about (and extending parallel to) the longitudinal axis 304. In the case of four stiffening wires 802, the stiffeners can be separated from each other by 90 degrees about the longitudinal axis 304. Similarly, three stiffening wires 802 may be separated by 120 degrees.

The pacing element 108 can include the insulation sleeve 412 surrounding the pacing element 108. More particularly, the insulation sleeve 412 can surround and contain the flexible conductor 502. For example, the insulation sleeve 412 may include a tubular sleeve or jacket having a lumen within which the flexible conductor 502 extends. The tubular structure can include a wall 804, e.g., an annular wall 804, defining the lumen within which the flexible conductor 502 is disposed. In an embodiment, the one or more stiffening wires 802 extend through the wall 804 of the insulation sleeve 412. More particularly, the stiffening wires 802 can be encapsulated by the wall 804 and, thus, may be disposed radially inward of an outer surface of the wall 804 and radially outward of an inner surface of the wall 804. The stiffening wires 802 embedded in the insulation sleeve 412, e.g., a polymer jacket, can impart stiffness to the overall structure, while the flexible conductor 502 can be highly flexible and therefore have high fatigue resistance.

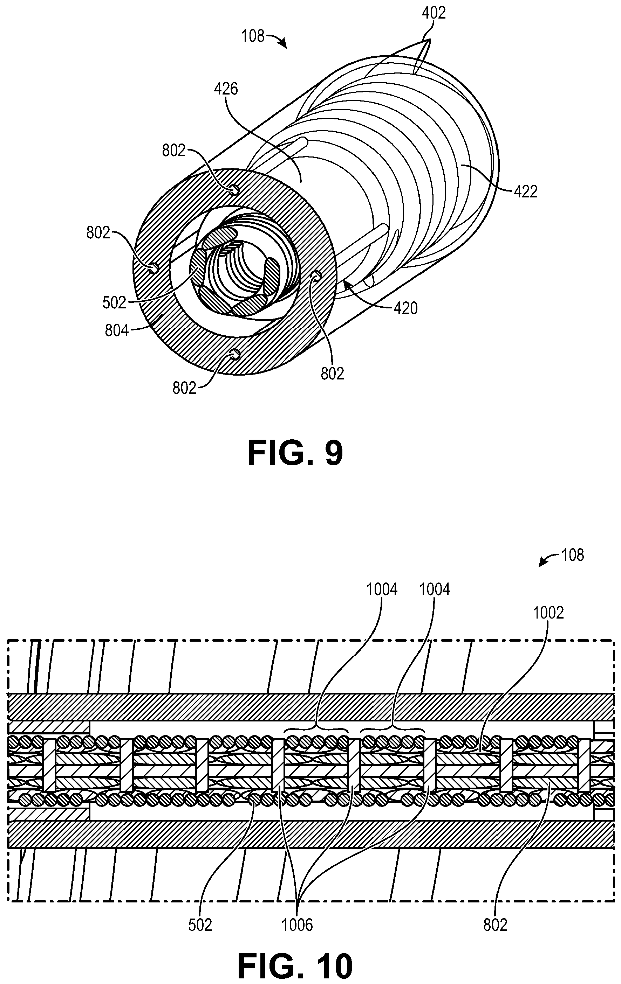

Referring to FIG. 9, a rear perspective view of a distal portion of a biostimulator having a stiffened pacing element is shown in accordance with an embodiment. A concentric relationship of the pacing element 108 components is shown. For example, the flexible conductor 502, which may be formed from a multi-filament coil, can be concentrically located within the annular wall 804 and the distal tip 420. Similarly, the stiffening wires 802 can be disposed symmetrically about the flexible conductor 502 such that the conductive coil is concentric with the cross-sectional profile of the combined stiffening elements. The helical electrode 422, which can extend distal to the flexible conductor 502 to the distal pacing tip 402, may be concentric with the flexible conductor 502 along the longitudinal axis 304.

It will be appreciated that the bending stiffness of the flexible conductor 502 may be varied, e.g., by varying the coil pitch as described above. The bending stiffness of the stiffening wires 802 may also vary. The variation may be over a length of the stiffening wires 802. For example, the wires may taper in the distal direction 408 to provide a reduction in cross-sectional area and, thus, bending stiffness in the distal direction 408. The variation may also be time-based. In an embodiment, the one or more stiffening wires 802 include a bioabsorbable material, such as a magnesium or tungsten material. Alternatively, the stiffeners can include bioabsorbable material, such as a bioabsorbable polymer, injected into channels within the insulation sleeve 412. Over time, the stiffeners can degrade, e.g., through bulk or surface degradation. For example, the pacing element 108 may include a path for fluid ingress to reach the bioabsorbable structures, and the fluid can cause degradation that leads to the stiffeners breaking up into smaller, disconnected pieces. Accordingly, a rigidity of the overall stiffening wire structure can reduce and the bending stiffness of the stiffeners can increase.

Referring to FIG. 10, a side view of a distal portion of a biostimulator having a stiffened pacing element is shown in accordance with an embodiment. The embodiment may incorporate a time-varying stiffness of the stiffening element component of the pacing element 108. More particularly, the pacing element 108 can include the flexible conductor 502 having a central lumen 1002 and a stiffening wire 802 extending along the longitudinal axis 304 through the central lumen 1002, which has a bending stiffness that varies over time.

As described above, a bending stiffness of the stiffening wire 802 (cable) can initially be greater than a bending stiffness of the flexible conductor 502. For example, the flexible conductor 502 can include a highly flexible coil, and the stiffening wire 802 may be a relatively rigid cable or rod extending through the central lumen 1002 along the longitudinal axis 304. The higher bending stiffness can support fatigue resistance of the pacing element 108. Similarly, the columnar structure of the stiffening wire 802 (or cable having several wires) can support tissue penetration, via column strength of the pacing element 108. The bending stiffness of the stiffening wire 802 may, however, reduce over time.

The stiffening wire 802 can have a segmented construction. For example, the stiffening wire 802 can include several wire segments 1004 contained within the central lumen 1002 of the flexible conductor 502. The wire segments 1004 may be stacked within the central lumen 1002. The stacking can place adjacent end faces of the segments against each other such that the segments form a segmented column. In an embodiment, the wire segments 1004 are joined by an adhesive. For example, adhesive joints 1006 can include adhesive potting of the end faces to connect the segments rigidly. The adhesive joints 1006, at least initially, can cause the segments to behave like a single, columnar stiffening wire 802.

In an embodiment, the segmented stiffening wire or cable can become more flexible over time. The adhesive used to form the adhesive joints 1006 can be a bioabsorbable adhesive. As the bioabsorbable adhesive degrades, the adhesive joints 1006 can crack and give way, allowing the segments to flex or move relative to each other. The disjointed segments can therefore form a more flexible stiffening wire structure, which has preferential articulation points at the degraded adhesive joints 1006. Bending stiffness of the stiffening wire 802 may therefore decrease, and may become lower than the bending stiffness of the flexible conductor 502, in an embodiment.

The bending and/or column stiffness of the stiffening wire 802 can be tuned. For example, the lengths of the wire segments 1004 may be varied to cause the overall structure to have a distally decreasing bending stiffness when the segments are disjointed. More particularly, a length of the wire segments 1004 may decrease in the distal direction 408, with more proximal segments being longer than more distal segments, to create a varied bending stiffness. The column strength of the stiffening wire 802 can remain high in both the initial state and the disjointed state because the end faces can abut each other when the pacing element 108 is compressed, and form a column structure that is longitudinally stiff. Accordingly, the segmented stiffening wire 802 can provide a structure that can penetrate tissue and which can resist bending initially, but can become more flexible over time to contribute to fatigue resistance.

Referring to FIG. 11, a perspective view of a header assembly of a biostimulator system is shown in accordance with an embodiment. A header assembly 1100 can include components described above, e.g., with respect to FIG. 4. For example, the header assembly 1100 can include the fixation element mount 406, the fixation element 106, and the pacing element 108. The pacing element 108 can extend longitudinally along a central axis, e.g., the longitudinal axis 403, distal to the fixation element 106. The pacing element 108 may support the helical electrode 422 to engage and pace target tissue.

In an embodiment, the pacing element 108 includes one or more stiffening strands 503 formed into a braid. More particularly the strands can be interlaced to form a stiffening braid 1102. The stiffening braid 1102 can have a tubular braid structure. For example, the tubular braid may be formed from biocompatible metallic or polymeric filaments, providing mechanical reinforcement and flexibility. The stiffening braid 1102 can provide stiffness to the overall pacing element structure, similar to the stiffening coil 504 described above. Accordingly, features of the stiffening coil 504 described above can apply similarly to the structure of the stiffening braid 1102. For example, the stiffening braid 1102 can surround the flexible conductor 502, which extends along the longitudinal axis 304 of the pacing element 108. Whereas the stiffening coil 504 can have one or more stiffening strands 503 revolving about the longitudinal axis 304 in a same direction, the stiffening strands 503 of the stiffening braid 1102 can revolve about the longitudinal axis in opposite directions.

The pacing element 108 can decouple stiffness from fatigue resistance. As described above, a bending stiffness of the stiffening strands 503 (or of the overall stiffening braid 1102 structure) may be greater than a bending stiffness of the flexible conductor 502. Accordingly, the stiffening braid 1102 can impart stiffness to promote tissue penetration and resilience, and the flexible conductor 502 can have good fatigue resistance.

In an embodiment, the stiffening braid 1102 and the flexible conductor 502 are encapsulated or contained within the insulation sleeve 412. The insulation sleeve 412 can have the structure described above. Alternatively, the insulation sleeve 412 can have a structure as described below. In any case, the insulation sleeve 412 can provide hermeticity, stiffness, and favorable metal ion oxidation (MIO) characteristics.

The pacing element 108 can include one or more additional layers surrounding the insulation sleeve 412. The additional layers can include an outer jacket 1104 surrounding the insulation sleeve 412. Material and size characteristics of the outer jacket 1104 can be selected to tailor a stiffness of the pacing element 108. Similarly, stiffness characteristics of the pacing element 108 can be further tuned by a strain relief 1106. The insulation sleeve 412, the outer jacket 1104, and/or the strain relief 1106 combine to form a composite structure surrounding the core assembly 410. The composite structure contributes to stiffness of the pacing element 108.

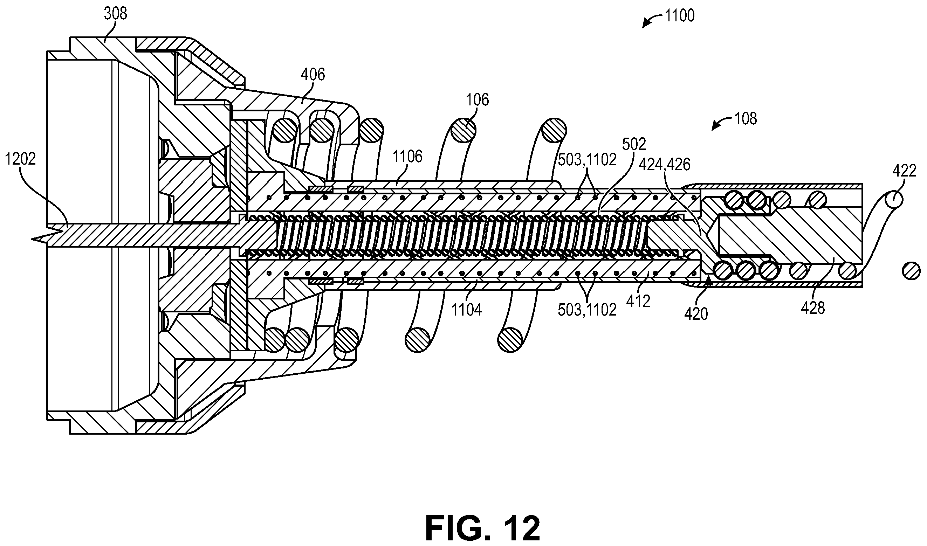

Referring to FIG. 12, a cross-sectional view of a header assembly of a biostimulator system is shown in accordance with an embodiment. In cross-section, certain relationships between components of the pacing element 108 are evident. The fixation element 106 can be mounted on, e.g., screwed into, the fixation element mount 406. Furthermore, the fixation element mount 406 can be mounted on the flange 308, which may be mounted on the housing 302. Accordingly, the fixation element 106 can be mounted on the housing 302 via the fixation element mount 406 and/or the flange 308.

The pacing element 108 can be mounted centrally within the fixation element 106. A proximal end of the pacing element 108, e.g., a proximal end of the flexible conductor 502, can be connected to a pacing pin 1202. Similarly, a distal end of the flexible conductor 502 can be connected to the distal tip 420, which the helical electrode 422 is mounted on. Accordingly, pacing pulses can be delivered distally from the pacing pin 1202 through the flexible conductor 502 to the helical electrode 422.

The flexible conductor 502 may have a lower stiffness than other components of the pacing element 108. For example, the flexible conductor 502 may be a thin coiled wire, as described above. The conductor may, as a result of such flexibility, have good fatigue resistance. For example, it has bene shown that a safety factor of the flexible conductor 502 when subjected to cyclic loading can be at least 3.0, e.g., the flexible conductor 502 can have a safety factor of 3.7.

The high fatigue resistance of the flexible conductor 502 can be provided by the thin, coiled wire described above. It will be appreciated, however, that similar fatigue resistance may be achieved by alternative flexible conductor structures. For example, the flexible conductor 502 may include a cable extending longitudinally along (or parallel to) the longitudinal axis 304 of the pacing element 108. The cable may include one or more thin wires, which can flex under side loads while conveying pacing pulses to the helical electrode 422.

In an embodiment, at least a portion of the core assembly 410 is encapsulated in the insulation sleeve 412. For example, the stiffening braid 1102 may be nested in a wall of the insulation sleeve 412. The stiffening braid 1102 can include several stiffening strands 503 interlaced, woven, etc. to form a patterned structure that provides stiffness to the pacing element 108. By way of example, the stiffening braid 1102 can include a braid of thin, elongated wires having a pic per inch (PPI) count in a range of 60-120. For example, the stiffening braid 1102 can have a 60 PPI count. Alternatively, the stiffening braid can have a 100 PPI count. The stiffening braid 1102 may be woven from thin metallic wires, e.g., may be formed from 0.001 to 0.003-inch diameter MP35N wires. For example, the metallic wires may be MP35N wires having a diameter of 0.0018-inch. The pattern may, for example, include a one-over-one-under structure. Such characteristics, although not limiting, have been shown to produce a pacing element having good fatigue characteristics.

In addition to imparting good fatigue characteristics, the stiffening braid 1102 can improve torqueability of the pacing element 108. The stiffening strands 503 of the braid, which revolve around the longitudinal axis 304 in different helical directions, can resist torsion in opposite directions. Accordingly, a torqueable structure is created. The torqueability may, for example, be comparatively better than the stiffening coil 504 that includes a one-directional helical stiffening strand 503.

In addition to having favorable fatigue and torqueability characteristics, the stiffening braid 1102 can impart stiffness and resilience to the pacing structure. The braid may have characteristics similar to those described with respect to the stiffening coil 504. For example, the stiffening strand construction may be altered to tune stiffness along the pacing element 108. Such tuning can include altering strand diameter, varying PPI count over a length of the pacing element, etc. Accordingly, the stiffening braid 1102 can be formed to provide one or more of bending stiffness or torsional stiffness to the pacing element.

In addition to stiffening the pacing element 108, the stiffening braid 1102 can impart resilience to the structure. The stiffening braid 1102 can be formed from material that experiences non-plastic deformation under bending. For example, the stiffening braid 1102 can be formed from a metal alloy that does not plastically deform under the stresses seen during operation. When the pacing element 108 is deflected under side loads during implantation, the stiffening braid 1102 can flex. However, when the side load is removed, the stiffening braid 1102 can cause the pacing element 108 to spring back to an undeflected state. Such resilience can be imparted whenever the pacing element 108 is deflected, allowing the pacing element to cyclically deform, e.g., under pulsatile conditions, without permanently deforming.

Optionally, the stiffening braid 1102 can perform an electrically conductive function. For example, the stiffening braid 1102 may connect to an electrode to perform an anodic or cathodic function during pacing. Accordingly, the stiffening braid 1102 may perform both mechanical and electrical functions of the pacing element 108.

The stiffening braid 1102 can be surrounded by and/or encapsulated within the insulation sleeve 412. In an embodiment, the insulation sleeve 412 contributes beneficial characteristics to the pacing element 108, such as stiffness and metal ion oxidation (MIO) resistance. The insulation sleeve 412 can be formed from a polymer having a bending stiffness that is higher than the flexible conductor 502. In an embodiment, the insulation sleeve 412 is formed from a polymer having a durometer of 90A on the Shore A scale, which is roughly equivalent to a durometer of 40D on the Shore D scale.

In an embodiment, the insulation sleeve 412 is formed from a material blend of polyurethane and silicone. More particularly, the insulation sleeve 412 can be formed from a polyurethane and silicone copolymer. The material blend, which combines beneficial characteristics of the constituents, has good MIO resistance. MIO is a chemical process where metal ions react with oxygen, leading to the formation of oxides that can degrade material properties. Resistance to MIO can be critical in medical devices such as biostimulators to promote longevity and reliability of components that are exposed to bodily fluids and varying environmental conditions. Enhanced MIO resistance in materials like the insulation sleeve 412 of the pacing element 108 can prevent premature wear of the stiffening braid 1102 and/or flexible conductor 502 and can maintain integrity of the biostimulator.

The pacing element 108 can include the outer jacket 1104 surrounding the insulation sleeve 412. The outer jacket 1104 can include a thin, tubular jacket of polymer shrouding the insulation sleeve 412. The outer jacket 1104 may stiffen the overall structure. For example, the outer jacket 1104 can be formed from a polymer having a higher stiffness than the insulation sleeve 412. By way of example, the outer jacket 1104 may be formed from a thermoplastic polyurethane material, e.g., Pellethane, having a stiffness in a range of 55D to 75D on the Shore D scale. Accordingly, the outer jacket material can be stiffer than the insulation sleeve material. The outer jacket 1104 may therefore be used to tailor a stiffness of the overall pacing element structure.

It will be appreciated that the outer jacket 1104 may be optional. An insulation sleeve 412 having sufficient stiffness may be used to attain a desired stiffness profile in the absence of the outer jacket 1104. More particularly, the insulation sleeve 412 may be formed from a material having a stiffness higher than 90A on the Shore A scale to achieve the desired stiffness profile. Nonetheless, the composite structure described above having the combined insulation sleeve 412 and outer jacket 1104 has been shown to provide a favorable stiffness profile with excellent MIO resistance.

In an embodiment, the pacing element 108 includes the strain relief 1106. The strain relief 1106 can include a polymeric sleeve having a stiffness similar or higher than the stiffness of the outer jacket 1104. The strain relief 1106 may, however, surround only a portion of the insulation sleeve 412. For example, the strain relief 1106 may surround a proximal portion of the outer jacket 1104 and a distal portion of the outer jacket 1104 may remain exposed distal to the strain relief 1106.

The composite structure provided by the insulation sleeve 412 and the outer jacket 1104 may be manufactured in various manners. The insulation sleeve 412 and the outer jacket 1104 may contain polyurethane. Accordingly, the components may bond well to each other. For example, the insulation sleeve 412 and the outer jacket 1104 may be formed as tubular components that are slid over each other and then reflowed to form a single, composite structure. Alternatively, the components may be co-extruded to form the single, composite structure. In any case, the composite structure can encapsulate the stiffening braid 1102 and contribute favorable stiffness and MIO resistance to the pacing element 108.

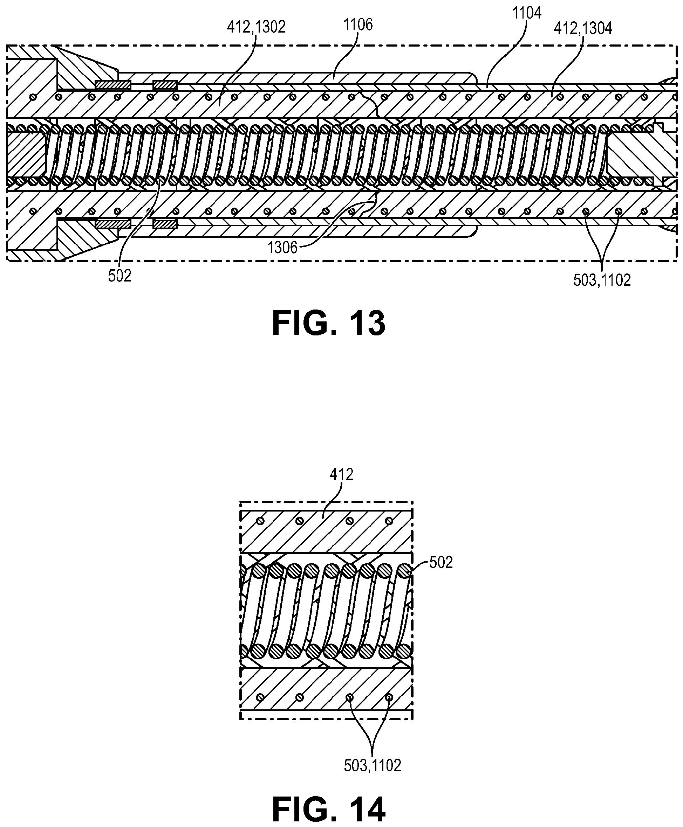

Referring to FIG. 13, a cross-sectional view of a pacing element of a biostimulator system is shown in accordance with an embodiment. The insulation sleeve 412 may be formed from several subcomponents having varying stiffnesses. In an embodiment, the insulation sleeve 412 includes a proximal sleeve component 1302 and a distal sleeve component 1304. The sleeve components may have similar cross-sectional dimensions. More particularly, the sleeve components can have a same inner and outer diameter. The sleeve components may, however, be formed from different durometers. The proximal sleeve component 1302 may be stiffer than the distal sleeve component 1304. For example, the proximal sleeve component 1302 can have a stiffness of 75D on the Shore D scale and the distal sleeve component 1304 can have a stiffness of 40D or 55D on the Shore D scale. The subcomponents can be formed from the same or different materials. The proximal sleeve component 1302 may, for example, be formed of Pellethane. The distal sleeve component 1304 may, for example, be formed of Pellethane or a polyurethane/silicone copolymer.

The proximal and distal components of the insulation sleeve 412 can be bonded in a reflowing operation. For example, a distal face of the proximal sleeve component 1302 can be pressed against a proximal face of the distal sleeve component 1304, and the components may be heated to cause the materials to flow and bond together. The faces can bond to each other at a seam 1306. The composite structure can provide a stiffness profile that decreases in a distal direction. The composite structure may be formed from different materials and components, however, a diameter of the insulation sleeve 412 can be consistent over an entire length of the pacing element 108.

Referring to FIG. 14, a cross-sectional view of a pacing element of a biostimulator system is shown in accordance with an embodiment. The pacing element 108 may not have the outer jacket 1104. A portion of the pacing element 108 is shown, e.g., a segment longitudinally between the strain relief 1106 and the distal tip 420. The segment can include the stiffening braid 1102 embedded in the insulation sleeve 412. In an embodiment, an outer surface of the insulation sleeve 412 is exposed to a surrounding environment. For example, as described above, the insulation sleeve 412 can be formed from a material that has sufficient stiffness and MIO resistance to perform the functions fulfilled by the composite structure of the outer jacket 1104 and the insulation sleeve 412 in the above embodiments. Accordingly, the pacing element 108 can have a structure that provides good torqueability, flexibility, MIO resistance, and resilience, while permitting the flexible conductor 502 to be very flexible and fatigue resistant.

The present invention is set out in the claims that follow.

## Claims

1. A biostimulator (100), comprising:
a housing (302) having a longitudinal axis (304) and containing an electronics compartment (306);
a fixation element (106) coupled to a distal housing end (309) of the housing (302), wherein the fixation element (106) extends about the longitudinal axis (304); and
a pacing element (108) coupled to the housing (302), wherein the pacing element (108) includes:
a flexible conductor (502) including a conductive cable (506) extending along the longitudinal axis (304), wherein the conductive cable (506) is a conductor coil;
an insulation sleeve (412) surrounding the pacing element (108), **characterized in that** the pacing element (108) further comprises:
a plurality of stiffening wires (802) disposed radially about the flexible conductor (502) and forms a stiffening braid (1102) extending through a wall (804) of the insulation sleeve (412), wherein a bending stiffness of the stiffening braid (1102) is greater than a bending stiffness of the flexible conductor (502); and
a helical electrode (422) coupled to the flexible conductor (502), wherein
the fixation element (106) includes a helical fixation element; and
the pacing element (108) is coupled to the distal housing end (309) of the housing (302).

2. The biostimulator of claim 1, wherein the bending stiffness of the stiffening braid (1102) decreases in a distal direction.

3. The biostimulator of claim 1 or 2, wherein a pitch of the plurality of stiffening wires (802) decreases in the distal direction.

4. The biostimulator of claim 3, wherein a diameter of the plurality of stiffening wires (802) decreases in the distal direction.

5. The biostimulator of any one of claims 1 to 4, wherein the plurality of stiffening wires (802) include a bioabsorbable material.

6. A biostimulator system (200), comprising:
a biostimulator transport system (202); and
the biostimulator (100) of any of claims 1-5 mounted on the biostimulator transport system (202).

## Patentansprüche

1. Biostimulator (100), umfassend:
ein Gehäuse (302), das eine Längsachse (304) aufweist und ein Elektronikfach (306) enthält;
ein Fixierungselement (106), das mit einem distalen Gehäuseende (309) des Gehäuses (302) gekoppelt ist, wobei sich das Fixierungselement (106) um die Längsachse (304) herum erstreckt; und
ein Herzschrittmacherelement (108), das mit dem Gehäuse (302) gekoppelt ist, wobei das Herzschrittmacherelement (108) Folgendes beinhaltet:
einen flexiblen Leiter (502), der ein leitendes Kabel (506) beinhaltet, das sich entlang der Längsachse (304) erstreckt, wobei das leitende Kabel (506) eine Leiterspule ist;
eine Isolierhülse (412), die das Herzschrittmacherelement (108) umgibt, **dadurch gekennzeichnet, dass** das Herzschrittmacherelement (108) ferner umfasst:
eine Mehrzahl von Versteifungsdrähten (802), die radial um den flexiblen Leiter (502) angeordnet ist und ein Versteifungsgeflecht (1102) bildet, das sich durch eine Wand (804) der Isolierhülse (412) erstreckt, wobei eine Biegesteifigkeit des Versteifungsgeflechts (1102) größer als eine Biegesteifigkeit des flexiblen Leiters (502) ist; und
eine schraubenförmige Elektrode (422), die mit dem flexiblen Leiter (502) gekoppelt ist, wobei das Fixierungselement (106) ein schraubenförmiges Fixierungselement beinhaltet; und das Herzschrittmacherelement (108) mit dem distalen Gehäuseende (309) des Gehäuses (302) gekoppelt ist.

2. Biostimulator nach Anspruch 1, wobei die Biegesteifigkeit des Versteifungsgeflechts (1102) in einer distalen Richtung abnimmt.

3. Biostimulator nach Anspruch 1 oder 2, wobei eine Teilung der Mehrzahl von Versteifungsdrähten (802) in der distalen Richtung abnimmt.

4. Biostimulator nach Anspruch 3, wobei ein Durchmesser der Mehrzahl von Versteifungsdrähten (802) in der distalen Richtung abnimmt.

5. Biostimulator nach einem der Ansprüche 1 bis 4, wobei die Mehrzahl von Versteifungsdrähten (802) ein bioabsorbierbares Material beinhaltet.

6. Biostimulatorsystem (200), umfassend:
ein Biostimulatortransportsystem (202); und
den Biostimulator (100) nach einem der Ansprüche 1 bis 5, der an das Biostimulatortransportsystem (202) montiert ist.

## Revendications

1. Biostimulateur (100), comprenant :
un boîtier (302) ayant un axe longitudinal (304) et contenant un compartiment d'électronique (306) ;
un élément de fixation (106) couplé à une extrémité de boîtier distale (309) du boîtier (302), dans lequel l'élément de fixation (106) s'étend autour de l'axe longitudinal (304) ; et
un élément de stimulation (108) couplé au boîtier (302), dans lequel l'élément de stimulation (108) inclut :
un conducteur flexible (502) incluant un câble conducteur (506) s'étendant le long de l'axe longitudinal (304), dans lequel le câble conducteur (506) est une bobine conductrice ;
un manchon d'isolation (412) entourant l'élément de stimulation (108), **caractérisé en ce que** l'élément de stimulation (108) comprend en outre :
une pluralité de fils de rigidification (802) disposés radialement autour du conducteur flexible (502) et forme une tresse de rigidification (1102) s'étendant à travers une paroi (804) du manchon d'isolation (412), dans lequel une rigidité en flexion de la tresse de rigidification (1102) est supérieure à une rigidité en flexion du conducteur flexible (502) ; et
une électrode hélicoïdale (422) couplée au conducteur flexible (502), dans lequel l'élément de fixation (106) inclut un élément de fixation hélicoïdal ; et l'élément de stimulation (108) est couplé à l'extrémité de boîtier distale (309) du boîtier (302).

2. Biostimulateur selon la revendication 1, dans lequel la rigidité en flexion de la tresse de rigidification (1102) diminue dans une direction distale.

3. Biostimulateur selon la revendication 1 ou la revendication 2, dans lequel un pas de la pluralité de fils de rigidification (802) diminue dans la direction distale.

4. Biostimulateur selon la revendication 3, dans lequel un diamètre de la pluralité de fils de rigidification (802) diminue dans la direction distale.

5. Biostimulateur selon l'une quelconque des revendications 1 à 4, dans lequel la pluralité de fils de rigidification (802) incluent un matériau bioabsorbable.

6. Système de biostimulateur (200), comprenant :
un système de transport de biostimulateur (202) ; et
le biostimulateur (100) selon l'une quelconque des revendications 1 à 5 monté sur le système de transport de biostimulateur (202).
